# EUROPÄISCHE PATENTSCHRIFT

(11) **EP 1 178 028 B1**
(45) Veröffentlichungstag und Bekanntmachung des Hinweises auf die Patenterteilung: **04.06.2003**
(21) Anmeldenummer: 01116166.8
(22) Anmeldetag: 04.07.2001
(51) Int. Cl.: C07C 7/08, C10G 7/08

(54) **Verfahren und Vorrichtung zur Erzeugung von Reinstaromaten**
Process and device for producing very pure aromatic compounds
Procédé et appareil pour la production d'hydrocarbures très purs

(30) Priorität: 05.08.2000 DE 10038318
(43) Veröffentlichungstag der Anmeldung: 06.02.2002
(73) Patentinhaber: Uhde GmbH, 44141 Dortmund (DE)
(72) Erfinder: Emmrich, Gerhard, 45133 Essen (DE); Ennenbach, Frank, 76344 Eggenstein (DE); Ranke, Uwe, Dr., 45128 Essen (DE); Gehrke, Helmut, Dr., 59269 Beckum-Vellern (DE)

(56) Entgegenhaltungen:
- US-A- 4 168 209

## Beschreibung

Die Erfindung betrifft ein Verfahren und eine Vorrichtung zur Erzeugung von hochreinen aromatischen Kohlenwasserstoffverbindungen, welche 6 bis 8 Kohlenstoffatome enthalten, und deren Gehalt an Verunreinigungen an organischen und anorganischen Verbindungen der Elemente Schwefel, Stickstoff, Sauerstoff und Chlor im ppb-Bereich liegt.

Die Freiheit von Verunreinigungen in Aromatenfraktionen ist ein seit langem bestehendes Bedürfnis der verarbeitenden Industrie. Hierbei wird gewünscht, dass die zur Weiterverwendung in chemischen Synthesen beabsichtigten aromatischen Kohlenwasserstoffe nur noch geringste Mengen an Verunreinigungen organischer und anorganischer Verbindungen der Elemente Schwefel, Stickstoff, Sauerstoff und Chlor enthalten dürfen, höchstens jedoch im ppb-Bereich, damit sensitiver und selektiver wirkende Katalysatorsysteme und hierbei besonders Katalysatoren auf Zeolithbasis zum Einsatz kommen können. So wird z. B. für die neuen Katalysatoren zur Ethylbenzolsynthese im Benzol ein maximaler Gehalt an organisch gebundenem Stickstoff von 30 ppb gefordert, wobei die Angabe ppb auf die Masse bezogen ist, wie auch bei allen folgenden ppm- und ppb-Angaben in dieser Schrift.

Reinaromaten werden üblicherweise durch Extraktivdestillation aus Kohlenwasserstoffgemischen wie vollhydriertem Pyrolysebenzin, Kokerei-Druckraffinat oder katalytischem Reformatbenzin gewonnen. Die Verunreinigungen der eingangs erwähnten Art mit Schwefel-, Sauerstoff-, Stickstoff-, Chlorverbindungen finden sich in den Aromaten nach solchen Extraktivdestillationen als Spurenverunreinigungen im ppm-Bereich. Sie resultieren zum einen aus Resten von Extraktions- oder Lösungsmitteln oder deren Zersetzung, zum anderen aus Stoffen, die mit dem Einsatzprodukt in die Anlage gelangt sind oder sich durch Reaktion in der Anlage gebildet haben.

Im bisherigen Stand der Technik werden solche verunreinigenden Bestandteile, wenn sie basisch reagieren, mit sauer aktivierter Bleicherde im Anschluss an die Extraktivdestillation aus den Aromaten entfernt. Eine solche Bleicherde-Behandlung bringt jedoch folgende, bekannte Nachteile mit sich:
- die Bleicherde hat nur eine begrenzte Beladungskapazität
- der Zeitpunkt des Durchbruches kann nicht exakt vorhergesagt werden
- es sollten mindestens stets zwei Bleicherdetürme parallel vorhanden sein
- die Bleicherde kann nicht regeneriert werden
- nach der Beladung muss die Bleicherde kohlenwasserstofffrei gedämpft werden
- die gedämpfte Bleicherde muss bergmännisch aus dem Turm abgebaut werden
- die Bleicherde muss geglüht werden, um etwaige verbliebene Kohlenwasserstoffe zu entfernen
- die geglühte Bleicherde muss deponiert werden.

Sauer reagierende, verunreinigende Bestandteile werden nach dem jetzigen Stand der Technik mit aktivierter Kohle, kaustischer Soda oder lonenaustauscherharzen aus den Kohlenwasserstoffen entfernt.

Bezüglich der Zugabe von Wasser in dampfförmige Kohlenwasserstoffe gibt die US 4,168,209 an, Wasser oberhalb der Extraktionsmittelzugabe in eine Destillationskolonne für die Extraktivdestillation einzugeben, das Kopfprodukt dieser Kolonne zu kondensieren und die sich ergebenden Phasen zu trennen. Im Unterschied zu der vorliegenden Erfindung dient diese Wasserzugabe jedoch nicht zur Reinigung der Kohlenwasserstoffe von unerwünschten Bestandteilen, sondern zur Minimierung der Extraktionsmittelverluste, hauptsächlich bereits innerhalb der Destillationskolonne, in die das Wasser eingeführt wird. Diese US 4,168,209 gibt auch nicht an, welche Reinheit erreicht werden könnte. Ein weiterer Unterschied der US 4,168,209 zu der vorliegenden Erfindung ist der Ort der Wasserzugabe, die vorliegende Erfindung sieht die Zugabestelle unmittelbar vor dem Kondensator vor und nicht in eine vorgeschaltete Kolonne, daher ist die vorliegende Erfindung unabhängig von Destillationskolonnen einsetzbar und es kann bei dem erfindungsgemäßen Reinigungsverfahren auch kein Wasser bzw. keine wässrige Lösung in eine Kolonne zurückfließen, wodurch die erfindungsgemäße Vorrichtung einen Nachteil der bekannten Vorrichtungen, bei denen der Ort der Wasserzugabe in der vorgeschalteten Kolonne vorgenommen wird, vermeidet.

Das erfindungsgemäße Verfahren besitzt die Aufgabe, die Nachteile der Bleicherdebehandlung zu vermeiden und ein wirtschaftliches Verfahren zur Erzeugung von Reinst-Aromatengemischen oder Reinstaromaten zur Verfügung zu stellen, die frei sind von kleinsten Verunreinigungen organischer Verbindungen der Elemente Schwefel, Stickstoff, Sauerstoff und Chlor bis in den ppb-Bereich hinein sowie eine verbesserte Vorrichtung dafür vorzuschlagen.

Es soll am Beispiel der Extraktivdestillation nach einem Verfahren, welches z. B. in der Schrift "MORPHYLANE® - Gewinnung hochreiner Aromaten", - eine von der Anmelderin Krupp Uhde GmbH im Mai 1997 in einer Auflage von 1000 Stück herausgegebene Druckschrift -, beschrieben ist und das stickstoffhaltige Extraktionsmittel N-Formylmorpholin (NFM) nutzt, näher erläutert werden. Das erfindungsgemäße Verfahren ist jedoch nicht auf Verfahren mit diesem Extraktionsmittel beschränkt, sondern ebenso anwendbar zusammen mit anderen Verfahren, die andere Extraktions- oder Lösungsmittel einsetzen, z. B. N-Methylpyrrolidon oder Tetramethylsulfon (Sulfolan®). Das Beispiel beschreibt die Gewinnung von hochreinem Benzol, ist jedoch uneingeschränkt für die Gewinnung von Aromaten mit bis zu 8 Kohlenstoffatomen und von Mischungen daraus einsetzbar und soll in diesem Sinne verstanden werden.

Das beispielgebende Extraktivdestillationsverfahren umfasst in der Regel eine Extraktivdestillationskolonne und eine nachgeschaltete Abtriebskolonne, beide können aber auch baulich zusammengefasst sein, wie die Schrift DE 198 49 651 lehrt. Außerdem kann der Extraktivdestillationskolonne eine Kolonne zur Vordestillation vorangeschaltet sein, um schwersiedende und leichtsiedende Bestandteile an verschiedenen Böden der Extraktivdestillationskolonne aufgeben zu können. In der Extraktivdestillationskolonne wird aus dem Einsatzprodukt, in diesem Beispiel einer sogenannten Benzolfraktion, welche aus einer Mischung aus Benzol und Nichtaromaten besteht, mittels eines selektiven Lösungsmittels, hier N-Formylmorpholin, das Benzol ausgewaschen. Die Nichtaromaten werden über Kopf gestrippt, während das Benzol zusammen mit dem Lösungsmittel zum Sumpf der Kolonne fließt. In der nachgeschalteten Abtriebskolonne werden Benzol und Lösungsmittel voneinander getrennt. Das abgetriebene Lösungsmittel sammelt sich im Sumpf der Abtriebskolonne und wird zum Wiedereinsatz zurück zum Kopf der Extraktivdestillationskolonne gepumpt. Das Benzol tritt dampfförmig am Kopf der Abtriebskolonne aus. Der Lösungsmittelrestgehalt beträgt im Durchschnitt 1 ppm N-Formylmorpholin bzw. das Hydrolyseprodukt Morpholin.

In diesen Benzoldampf wird in einer Mischzone direkt eine wässrige Lösung dispers eingebracht, zum Beispiel eingedüst. Die Lösungswassermenge, bezogen auf den Benzoldampf, kann zwischen 1 Gew.% und 20 Gew.% betragen, bevorzugt sind 5 Gew.%. Ein Teil der wässrigen Lösung wird dabei verdampft. Dadurch wird dem Benzoldampf Wärme entzogen, was bewirkt, dass ein Teil des Benzols aus der Dampfphase kondensiert und sich mit den Tröpfchen der eingedüsten wässrigen Lösung intensiv mischt, wobei ein erster Teil der unerwünschten Bestandteile aus der Benzolphase in die wässrige Phase, in denen sie sich leichter lösen, entsprechend dem Verhältnis ihrer Löslichkeiten überwechseln.

Dadurch entsteht ein zweiphasiger, nämlich mit Tröpfchennebel beladener Dampf. Seine Gasphase enthält im Wesentlichen den Dampf des eingesetzten Benzols sowie Wasserdampf. Seine Flüssigphase enthält überwiegend den eingedüsten Tröpfchennebel des Lösungswassers mit den darin gelösten verunreinigenden Bestandteilen. Dass die Flüssigphase nicht hauptsächlich aus Benzol, welches einen niedriger liegenden Siedepunkt als Wasser aufweist, besteht, liegt daran, dass die Verdampfung der Wassertröpfchen bei den Temperaturen im Bereich der Siedetemperatur der beteiligten Kohlenstoffverbindung ein relativ langsamer Vorgang ist und die Verweilzeit der Wassertröpfchen relativ dazu kurz ist. Wird jedoch die wässrige Lösung in einen zu reinigenden Kohlenwasserstoffeinsatz eingedüst, der z. B. im Wesentlichen aus Toluol mit einem Siedepunkt von 110 °C besteht oder aber der z. B. vorwiegend aus einem Gemisch des Ethylbenzols mit Xylolen besteht, welches Gemisch einen Siedebereich von 131 °C bis zu 144 °C aufweist, so wird der zweiphasige Dampf eine Temperatur oberhalb des Siedepunktes des Wassers annehmen und somit wird in diesem Fall der Kohlenwasserstoffanteil im Tröpfchennebel überwiegen. Der mit Tröpfchennebel beladene Dampf wird aus der Mischzone unmittelbar in eine Kondensationszone übergeleitet. In der Kondensationszone werden beide Phasen mit Kühlflächen in Kontakt gebracht, woran sie gemeinsam niedergeschlagen und in eine Kondensat-Flüssigkeit gewandelt werden, die als Emulsion der einen in der anderen Flüssigkeit anfällt.

In der Kondensationszone findet abermals eine intensive Vermischung der beiden Phasen statt und bewirkt, dass der in der Benzolphase verbliebene, andere Teil der verunreinigenden Bestandteile aus der Benzolphase in die wässrige Phase - in welcher wässrigen Phase der verbliebene andere Teil sich leichter löst - eben deshalb entsprechend dem Verhältnis ihrer Löslichkeiten bei optimal gering gehaltenem Stoffübergangswiderstand überwechseln kann. Die in der Kondensationszone gebildete Kondensatflüssigkeit besteht aus der einen, im Wesentlichen Benzol enthaltenden Teilflüssigphase und einer anderen, im Wesentlichen das Lösungswasser enthaltenden Teilflüssigkeitsphase. Die Kondensatflüssigkeit wird aus der Kondensationszone abgezogen und in eine Abscheidezone eingeleitet.

In der Abscheidezone wird die eine, im Wesentlichen Benzol enthaltende Teilflüssigphase von der anderen, im Wesentlichen das Lösungswasser mit den darin gelösten verunreinigenden Bestandteilen enthaltenden Teilflüssigphase abgetrennt. Die Abtrennung der einen Teilflüssigkeit von der anderen Teilflüssigkeit geschieht mittels Ausnutzung des unterschiedlichen spezifischen Gewichts beider Teilflüssigphasen, z. B. durch Schwerkraft oder Zentrifugalkraft oder andere vergleichbare Mittel. Daher ist die vorliegende Erfindung dahingehend ausgestaltet, dass die Abtrennung der einen, im Wesentlichen gereinigtes Benzol enthaltenden Teilflüssigphase der Emulsion von der anderen, im Wesentlichen Lösungswasser enthaltenden Teilflüssigphase der Emulsion mittels eines Wasserabscheiders erfolgt.

Die Benzolphase ist gereinigt und muss, falls ihre Wasserfreiheit für die spätere Verwendung gefordert wird, noch getrocknet werden. Die wässrige Phase wird üblicherweise, aber nicht notwendigerweise, in zwei Teilströme aufgespalten: Der eine Teilstrom wird biologisch gereinigt und entsorgt. Der andere Teilstrom wird zur Eindüsungsstelle zurückgeführt und bildet somit einen Kreislauf. Das Verhältnis der beiden Teilströme ergibt sich aus dem Gehalt der gelösten Schadstoffe und der Ansprüche an die Reinheit des Benzol-Produkts im einzelnen Anwendungsfall. Der ausführende Fachmann wird hierzu Laborversuche anstellen. Hierbei kann sich im Grenzfall auch ergeben, dass nur reines Frischwasser eingedüst werden darf, und dass die im Wasserabscheider abgetrennte wässrige Lösung vollständig entsorgt werden muss. Daher geschieht eine weitere Ausgestaltung der vorliegenden Erfindung, bei der zumindest ein Teil des aus der Emulsion abgetrennten Lösungswassers in einem Kreislauf rückgeführt wird und in die besagten Mischzone als Bestandteil der wässrigen Lösung dispers eingebracht wird und bei der mit dem verbliebene Rest des aus der Emulsion abgetrennten Lösungswassers die besagten verunreinigenden Beständteile aus dem Lösungswasserkreislauf ausgeschleust und ihrer Entsorgung zugeführt werden.

Der vorteilhafte Einsatz des erfindungsgemäßen Verfahrens soll am Beispiel einer Extraktivdestillation zur Erzeugung hochreinen Benzols mittels des stickstoffhaltigen Extraktionsmittels N-Formylmorpholin näher erläutert werden, wobei der Einsatz selbstverständlich nicht auf Extraktivdestillationen oder auf die Entfernung von N-Formylmorpholin oder auf die Reinigung von Benzol beschränkt ist.

Das beispielgebende Extraktivdestillationsverfahren umfasst in der Regel mindestens zwei Kolonnen, eine Extraktivdestillationskolonne und eine nachgeschaltete Abtriebskolonne, beide können auch als Trennwandkolonne bzw. gradierende Kolonne baulich zusammengefasst sein. In der ersten Kolonne, der Extraktivdestillationskolonne, wird aus dem Einsatzprodukt, in diesem Falle einer Benzolfraktion, mittels eines selektiven Lösungsmittels, hier N-Formylmorpholin, das Benzol ausgewaschen. Die Nichtaromaten werden über Kopf gestrippt, während das Benzol zusammen mit dem Lösungsmittel zum Sumpf der Kolonne fließt. In der zweiten Kolonne, der Abtriebskolonne, werden Benzol und Lösungsmittel voneinander getrennt. Das abgetriebene Lösungsmittel sammelt sich im Sumpf der Abtriebskolonne und wird zum Wiedereinsatz zurück zum Kopf der Extraktivdestillationskolonne gepumpt. Das Benzol tritt dampfförmig am Kopf der Abtriebskolonne aus. Beim bisherigen Stand der Technik wird es dann kondensiert und gesammelt im Rückflussbehälter, von wo ein Teil als Rückfluss zur Abtriebskolonne gepumpt wird. Der Rest des Benzols wird aus dem Rückflussbehälter als Fertigprodukt zur Anlagengrenze gefördert. Der Lösungsmittelrestgehalt im herkömmlichen Stand der Technik beträgt im Durchschnitt 1 ppm ( entspricht 1000 ppb), N-Formylmorpholin (NFM), oder das Hydrolyseprodukt Morpholin, wobei diese beiden Stoffe zu etwa 1/7, bezogen auf ihre Masse, aus Stickstoff bestehen.

Das erfindungsgemäße Verfahren ermöglicht es, den Stickstoffgehalt im Fertigprodukt durch Eindüsen von Lösungswasser, gegebenenfalls zusammen mit Ameisensäure, welche gemeinsam in den Dampfstrom des Benzols vom Kopf der Abtriebskolonne unmittelbar vor dem Kondensator eingedüst werden, überraschenderweise auf einen Gehalt von kleiner 30 ppb zu verringern. Dies liegt daran, dass der Verteilungsfaktor im Dreistoffsystem NFM/Morpholin-Benzol-Wasser 30-mal größer für NFM/Morpholin-Wasser als für NFM/Morpholin-Benzol ist. Die Löslichkeit von Wasser im Benzol und von Benzol im Wasser ist sehr gering (bei 50 °C 1,3 g Benzol/1000 g Wasser und 1,56 g Wasser/1000 g Benzol). Daher findet im Anschluss an die intensive Vermischung eine Phasentrennung statt und das Lösungsmittel NFM befindet sich in der wässrigen Phase. Das erfindungsgemäße Verfahren besitzt daher den überzeugenden Vorteil, mit einfachen Mitteln die Herstellung eines hochreinen Produktes verfügbar zu machen.

Mit einer anderen Ausgestaltung der vorliegenden Erfindung kann die Produktreinheit über das vorerwähnte Maß hinaus erhöht werden, indem dass zumindest ein Teil der in die Mischzone dispers eingebrachten wässrigen Lösung aus Frischwasser besteht.

In weiterer Ausgestaltung der vorliegenden Erfindung wird die im Kreis geführte wässrige Lösung durch Zusatz einer Säure, beispielsweise Ameisensäure, auf einen pH-Wert geringfügig oberhalb 7, etwa bei 7,5 eingestellt, um die bereits im Kreislaufwasser vorhandenen Stickstoffverbindungen als Salz aus dem Lösungsgleichgewicht zu entfernen. Hierzu wird der in die Mischzone dispers eingebrachten wässrigen Lösung eine Säure beigemischt. Das erfindungsgemäße Verfahren wird hierzu im Einzelnen so ausgestaltet, dass als zur wässrigen Lösung beigemischte Säure die Ameisensäure eingesetzt wird.

In einer anderen Ausgestaltung der vorliegenden Erfindung ist vorgesehen, dass die nach der Säurezugabe in der wässrigen Lösung ausgefällten Salze durch Abscheidemittel aus der wässrigen Lösung entfernt werden.

Weiterhin besitzt das erfindungsgemäße Verfahren eine Ausgestaltung, nach der die Säurezugabe pH-Wert-geregelt erfolgt.

Bei der vorliegenden Erfindung sieht eine spezielle Verfahrensausgestaltung vor, die wässrige Lösung vor ihrem dispersen Einbringen in die Mischzone zu kühlen.

Auch besitzt die vorliegende Erfindung noch eine Verfahrensausgestaltung, bei der die in der Kondensationszone entstandene Kondensat-Emulsion vor ihrer Einleitung in die Abscheidezone unterkühlt wird.

Mit der vorliegenden Erfindung wird zugleich eine Vorrichtung zur Durchführung des erfindungsgemäßen Verfahrens bereitgestellt. Wie voranstehend beschrieben, sieht das erfindungsgemäße Verfahren vor, die wässrige Lösung unmittelbar vor dem Kondensator in den Benzoldampf dispers einzubringen, z. B es einzudüsen. Als besonders wirkungsvoll in Bezug auf die erzielbaren Produktreinheiten hat es sich gezeigt, die Mischzone und die Kondensationszone unter Vermeidung irgend einer zwischengeschalteten Überleitungsstrecke direkt miteinander in einer integralen Apparatekonstruktion zu verbinden. So weist eine Ausgestaltung der erfindungsgemäßen Vorrichtung einen Einzelapparat auf, in welchem die Mischzone und die Kondensationszone in einem beide Zonen einhüllenden gemeinsamen Raum angeordnet sind und welcher Raum von dem Mantel des Einzelapparates integrierend umgrenzt wird.

Das Verfahren eignet sich darüber hinaus auch zur nachträglichen Installation in bestehende Anlagen, da überwiegend die Rückflussbehälter in Fraktionierungen, Extraktionen und Extraktivdestillationen mit Wasserabscheidevorrichtungen ausgestattet sind oder sich mit geringem Aufwand damit ausstatten lassen.

Das anfallende, von den Verunreinigungen befreite Reinprodukt fällt wassergesättigt an (Wasser im Benzol bei 50 °C 1,56 g/1000 g). Muss das Reinprodukt wegen nachfolgender Synthesen wasserfrei sein, so lässt sich leicht eine destillative oder adsorptive Trocknung anschließen.

Das erfindungsgemäße Verfahren wird in der beiliegenden Zeichnung beispielsweise näher beschrieben.

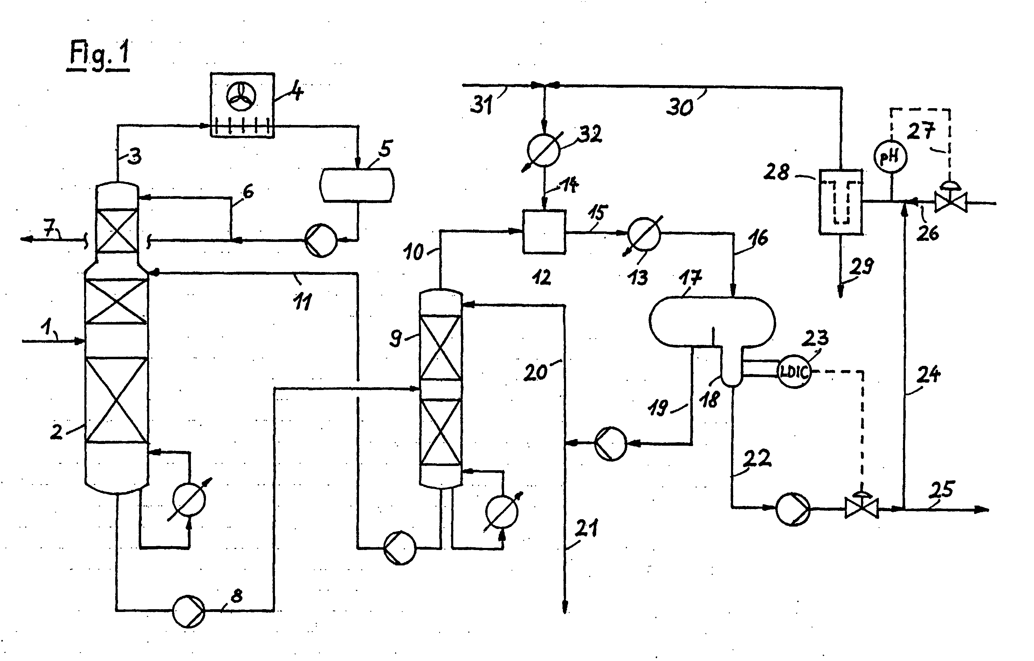

- Figur 1: zeigt das Verfahrensfließbild einer Anlage zur Herstellung von Reinstbenzol.

Die Reinigung der Einsatzfraktion 1 wird durch Extraktivdestillation mittels zweier Kolonnen, der Extraktivdestillationskolonne 2 und der Abtriebskolonne 9, vorgenommen. Als Lösungsmittel wird N-Formylmorpholin verwendet. Hierbei wird die Einsatzfraktion, die sowohl nichtaromatische als auch aromatische Verbindungen enthält, über die Leitung 1 in die Extraktivdestillationskolonne 2 gegeben. Bei der Einsatzfraktion kann es sich um unterschiedliche Benzol, Toluol und Xylole enthaltende Kohlenwasserstoffgemische, wie zum Beispiel Kokerei-Benzol-Druckraffinat, Pyrolysebenzin oder Reformatbenzin, handeln. In der Extraktivdestillationskolonne 2, die mit Böden oder sonstigen Einbauten versehen und auch als gradierende Kolonne ausgebildet sein kann, erfolgt die Abtrennung der Nichtaromaten von den Aromaten, wobei das erforderliche Lösungsmittel (z. B. N-Formylmorpholin) über die Leitung 11 auf die Extraktivdestillationskolonne 2 aufgegeben wird. Die Aromaten werden dabei zusammen mit dem Lösungsmittel als Extrakt aus dem Sumpf der Extraktivdestillationskolonne 2 abgezogen und gelangen über die Leitung 8 in die Abtriebskolonne 9, während gleichzeitig die Nichtaromaten dampfförmig über Kopf via Leitung 3 abgezogen, im Luftkühler 4 kondensiert, im Rückflussbehälter 5 gesammelt und zum einen als Rückfluss über die Leitung 6 in die Extraktivdestillationskolonne 2 zurückgeführt und zum anderen über die Leitung 7 ihrer weiteren Behandlung zugeführt werden.

Das von der Extraktivdestillationskolonne 2 kommende Benzol-Lösungsmittel-Gemisch wird über die Leitung 8 in die Abtriebskolonne 9 gegeben. In der Abtriebskolonne 9 erfolgt die Abtrennung des Benzols vom Lösungsmittel, wobei das Benzol als dampfförmiges Kopfprodukt über die Leitung 10 aus der Abtriebskolonne 9 abgezogen wird. Das Kopfprodukt enthält die Verunreinigungen, z.B. Spuren des Lösungsmittels. Das von Benzol befreite Lösungsmittel wird aus dem Sumpf der Abtriebskolonne 9 entfernt und über die Leitung 10 zwecks Wiederverwendung zur Extraktivdestillationskolonne zurückgeführt.

Unmittelbar vor Eintritt der Benzoldämpfe in den Kondensator 13 wird in der Mischzone 12, die als Sprühvorrichtung ausgeführt ist, eine wässrige Lösung 14 eingedüst. Als wässrige Lösung 14 ist eine Mischung aus vollentsalztem Wasser, Wasserdampfkondensat, zurückgeführter wässriger Lösung und Ameisensäure vorgesehen. Diese wässrige Lösung verdampft zum Teil in der Mischzone 12, die hierbei dem Benzoldampf entzogene Energie bewirkt eine Teilkondensation von Benzol in der Leitung 15. Im anschließenden Kondensator 13 wird die verdampfte wässrige Lösung zusammen mit dem restlichen Benzoldampf kondensiert und fällt zusammen mit den bereits kondensierten Tröpfchen im Kondensator 13 aus. Sowohl bei der Teilkondensation nach der Eindüsungsstelle in der Mischzone 12 für die wässrige Lösung als auch im Kondensator 13 findet eine intensive Mischung von Benzol und wässriger Lösung statt. Dabei wäscht die wässrige Lösung aus dem Kondensierten den größten Teil der Verunreinigungen aus.

Das kondensierte Benzol fließt mit der wässrigen Lösung über die Leitung 16 in den Rückflussbehälter 17, der mit einer Wasserabscheidevorrichtung 18 ausgestattet ist. Das gereinigte Benzol wird über die Leitung 19 abgezogen und zum einen als Rückfluss über die Leitung 20 in die Abtriebskolonne 9 geleitet und zum anderen als Produkt-Benzol 21 aus der Reinigungsvorrichtung abgezogen. Im Rückflussbehälter 17 löst sich der Rest der Verunreinigungen in der wässrigen Lösung. Aus der Abscheidevorrichtung 18 wird die wässrige Lösung 22 über eine Trennschichtregelung 23 ausgeschleust und teilweise über die Leitung 24 zurück zur Eindüsung vor den Kondensator gepumpt. Über die Leitung 25 wird ein anderer Teil der wässrigen Lösung 22 als Abwasser zur Aufarbeitung in eine biologische Kläranlage abgeführt. Das Verhältnis dieser beiden Ströme in den Leitungen 24 und 25 ergibt sich aus dem Gehalt der in der wässrigen Lösung gelösten Schadstoffe und der Ansprüche an die Reinheit des Benzols im Einzelfall. Zu beachten sind hierbei die Löslichkeitsgleichgewichte der Verunreinigungen sowohl für die Phase der wässrigen Lösung als auch die des Benzols. Der ausführende Fachmann wird hierzu im Einzelfall Laborversuche anstellen. Hierbei kann sich im Einzelfall auch ergeben, dass nur reines Frischwasser über die Leitung 14 eingedüst werden darf, und dass das die im Rückflussbehälter 17 abgetrennte wässrige Lösung 22 vollständig entsorgt werden muss.

Der zur Eindüsung bestimmten wässrigen Lösung in der Leitung 24 wird zur Einstellung eines pH-Wertes im Bereich von 7 bis 7,5 Ameisensäure 26 zugegeben, wobei die Dosierung über eine pH-Wert-Regelungseinrichtung 27 erfolgt. Durch die pH-Wert-Erniedrigung fällt ein Lösungsmittelsalz 29 aus, welches im Filter 28 aus der wässrigen Lösung ausgeschieden wird. Hierdurch wird eine Anreicherung von bereits abgeschiedenen Verunreinigungen in der wässrigen Lösung verhindert. Über die Leitung 30 wird die wässrige Lösung ergänzt um Wasser aus der Frischwasserleitung 31, um den Kreislauf um dasjenige Wasser aufzufüllen, welches gelöst im Produkt-Benzol 21 oder als Abwasser über die Leitung 25 die Reinigungseinrichtung verlässt. Um den Kondensationseffekt in der Mischzone 12 zu verstärken, kann die wässrige Lösung bei Bedarf im Wasserkühler 32 gekühlt werden.

### Bezugszeichenlegende:

- 1: Leitung für Einsatzfraktion
- 2: Extraktivdestillationskolonne
- 3: Leitung für Nichtaromaten-Dampf
- 4: Luftkühler
- 5: Rückflussbehälter
- 6: Leitung für Rückfluss
- 7: Nichtaromaten
- 8: Leitung für Benzol-Lösungsmittelgemisch
- 9: Abtriebskolonne
- 10: Leitung für dampfförmiges Benzol
- 11: Leitung für Lösungsmittel
- 12: Mischzone
- 13: Kondensator
- 14: wässrige Lösung
- 15: Leitung
- 16: Leitung für Kondensat
- 17: Rückflussbehälter
- 18: Wasserabscheidevorrichtung
- 19: Leitung für gereinigtes Benzol
- 20: Leitung für Rückfluss
- 21: Produkt-Benzol
- 22: wässrige Lösung
- 23: Trennschichtregelung
- 24: Leitung für Kreislauflösung
- 25: Leitung für Abwasser
- 26: Ameisensäure
- 27: pH-Wert-Regelungseinrichtung
- 28: Filter
- 29: Lösungsmittelsalz
- 30: Leitung
- 31: Frischwasserleitung
- 32: Wasserkühler

## Patentansprüche

1. Verfahren zur Trennung von Kohlenwasserstoffgemischen, welche aromatische Verbindungen mit 6 bis 8 Kohlenstoffatomen enthalten, durch Extraktivdestillation mit selektiven Lösungsmitteln bzw. Lösungsmittelgemischen, wobei das Einsatzprodukt in den mittleren Teil und das verwendete Lösungsmittel bzw. Lösungsmittelgemisch in den oberen Teil einer der Extraktivdestillation dienenden Kolonne eingeleitet wird und die im Lösungsmittel-Kohlenwasserstoffgemisch leichtersiedenden Kohlenwasserstoffe des Einsatzproduktes über Kopf aus besagter, der Extraktivdestillation dienenden Kolonne abgezogen werden, während die schwerer siedenden Kohlenwasserstoffe des Einsatzproduktes zusammen mit der Hauptmenge des Lösungsmittels als Sumpfprodukt der der Extraktivdestillation dienenden Kolonne anfallen und das Sumpfprodukt aus der der Extraktivdestillation dienenden Kolonne in eine dem Abtreiben des Lösungsmittels dienenden, nachgeschalteten, aber nicht notwendigerweise baulich von der der Extraktivdestillation dienenden Kolonne getrennt ausgeführten Kolonne überführt wird, **dadurch gekennzeichnet, dass** in das dampfförmige Kopfprodukt aus der dem Abtreiben des Lösungsmittels dienenden Kolonne in einer Mischzone eine wässrige Lösung dispers eingebracht wird, der dadurch gebildete zweiphasige, mit Tröpfchennebel beladene Dampf unmittelbar in eine Kondensationszone übergeleitet wird, worin er mit beiden Phasen gemeinsam niedergeschlagen wird und die entstandene Kondensat-Emulsion in eine Abscheidezone eingeleitet wird, in der die im Wesentlichen gereinigten Kohlenwasserstoff enthaltende eine Teilflüssigphase der Emulsion von der im Wesentlichen Lösungswasser mit den darin gelösten verunreinigenden Bestandteilen enthaltenden anderen Teilflüssigphase der Emulsion abgetrennt wird.

2. Verfahren nach Anspruch 1, **dadurch gekennzeichnet, dass** die Abtrennung der einen, im Wesentlichen gereinigten aromatische Kohlenwasserstoffe enthaltenden Teilflüssigphase der Emulsion von der anderen, im Wesentlichen Lösungswasser enthaltenden Teilflüssigphase der Emulsion mittels eines Wasserabscheiders erfolgt.

3. Verfahren nach einem der vorangegangenen Ansprüche 1 oder 2, **dadurch gekennzeichnet, dass** zumindest ein Teil des aus der Emulsion abgetrennten Lösungswassers in einem Kreislauf rückgeführt wird und in die besagte Mischzone als Bestandteil der wässrigen Lösung dispers eingebracht wird und mit dem verbliebenen Rest des aus der Emulsion abgetrennten Lösungswassers die besagten verunreinigenden Bestandteile aus dem Lösungswasserkreislauf ausgeschleust und ihrer Entsorgung zugeführt werden.

4. Verfahren nach einem der vorangegangenen Ansprüche 1 bis 3, **dadurch gekennzeichnet, dass** zumindest ein Teil der in die Mischzone dispers eingebrachten wässrigen Lösung aus Frischwasser besteht.

5. Verfahren nach einem der vorangegangenen Ansprüche 1 bis 4, **dadurch gekennzeichnet, dass** der in die Mischzone dispers eingebrachten wässrigen Lösung eine Säure beigemischt wird.

6. Verfahren nach Anspruch 5, **dadurch gekennzeichnet, dass** die der wässrigen Lösung beigemischte Säure Ameisensäure ist.

7. Verfahren nach Anspruch 5 oder 6, **dadurch gekennzeichnet, dass** die nach der Säurezugabe in der wässrigen Lösung ausgefällten Salze durch Abscheidemittel aus der wässrigen Lösung entfernt werden.

8. Verfahren nach Anspruch 5 bis 7, **dadurch gekennzeichnet, dass** die Säurezugabe pH-Wert-geregelt erfolgt.

9. Verfahren nach einem der vorangegangenen Ansprüche 1 bis 8, **dadurch gekennzeichnet, dass** die wässrige Lösung vor ihrem dispersen Einbringen in die Mischzone gekühlt wird.

10. Verfahren nach einem der vorangegangenen Ansprüche 1 bis 9, **dadurch gekennzeichnet, dass** die in der Kondensationszone entstandene Kondensat-Emulsion vor ihrer Einleitung in die Abscheidezone unterkühlt wird.

11. Vorrichtung zur Durchführung des Verfahrens nach mindestens einem der vorangegangenen Ansprüchen 1 bis 10, **dadurch gekennzeichnet, dass** die Mischzone und die Kondensationszone in einem beide Zonen einhüllenden gemeinsamen Raum angeordnet sind und dieser Raum von dem Mantel einer Einzelapparatur integrierend umgrenzt wird.

## Claims

1. Process for the separation of hydrocarbon mixtures containing aromatic compounds with 6 to 8 carbon atoms by extractive distillation with the aid of selective solvents or solvent mixtures, the feed product entering into the central part and the solvent being fed into the upper part of a column for extractive distillation, the lower boiling hydrocarbons of the feed product in the solvent/hydrocarbon mixture being withdrawn at the head of said column used for extractive distillation, while the higher boiling hydrocarbons of the feed product are withdrawn together with the main portion of the solvent from the bottom of the extractive distillation column, the bottom product from the extractive distillation column being piped to a column used for stripping the solvent and arranged downstream of the column for extractive distillation, the column for stripping the solvent not necessarily being separated from the column for extractive distillation,
**characterised in that** an aqueous solution is dispersed in a mixing zone and added to the vaporous head product from the column used for stripping the solvent, the two-phase vapour thus formed and laden with a mist of droplets being directly sent to a condensation zone in which it is precipitated simultaneously with both phases, and the condensate emulsion thus formed being fed to the separation zone, in which the one partial liquid phase of said emulsion primarily containing purified hydrocarbon is separated from the other partial liquid phase primarily containing solution water with the impurities dissolved therein.

2. Process according to claim 1,
**characterised in that** the separation of the partial liquid phase of the emulsion, which primarily contains purified aromatic hydrocarbons, from the other partial liquid phase of the emulsion, which primarily contains solution water, is accomplished by means of a water separating device.

3. Process according to any one of the preceding claims 1 or 2,
**characterised in that** at least part of the solution water separated from the emulsion is recycled and dispersed as part of the aqueous solution into said mixing zone and that the impurities are eliminated from the solution water cycle with the remaining part of the solution water separated from the emulsion and sent to disposal facilities.

4. Process according to any of the preceding claims 1 to 3,
**characterised in that** at least part of the aqueous solution dispersed into the mixing zone consists of clean water.

5. Process according to any of the preceding claims 1 to 4,
**characterised in that** an acid is admixed to the aqueous solution dispersed into the mixing zone.

6. Process according to claim 5 above,
**characterised in that** the acid admixed to the aqueous solution is formic acid.

7. Process according to any one of the preceding claims 5 or 6,
**characterised in that** the salts precipitated after admixing acid to the aqueous solution are removed from the aqueous solution with the aid of precipitants.

8. Process according to any of the preceding claims 5 to 7,
**characterised in that** the acid admixture is pH-controlled.

9. Process according to any of the preceding claims 1 to 8,
**characterised in that** the aqueous solution is cooled prior to being dispersed into the mixing zone.

10. Process according to any of the preceding claims 1 to 9,
**characterised in that** the condensate emulsion that forms in the condensation zone is supercooled prior to being fed to the separation zone.

11. Facility for the performance of the process according to at least one of the preceding claims 1 to 10,
**characterised in that** the mixing zone and the condensation zone are arranged in a common space enveloping the two zones and that this space is enclosed in the shell of a single apparatus.

## Revendications

1. Procédé pour la séparation de mélanges d'hydrocarbures qui ont une teneur en composants aromatiques de 6 à 8 atomes de carbone, moyennant la distillation extractive qui a lieu à l'aide de solvants sélectifs ou de mélanges de solvants, dans laquelle le produit mis en oeuvre est introduit dans la partie centrale d'une colonne à distillation extractive et le dit solvant ou mélange est envoyé vers la partie supérieure de la dite colonne; les hyrocarbures légers contenus dans le dit produit et présents dans le mélange de solvant et d'hydrocarbures sont soutirés à la tête de la colonne à distillation extractive, tandis que la fraction plus lourde du produit mis en oeuvre est évacuée avec la plus grande partie du solvant au niveau du fond de la dite colonne; le produit du bas de colonne à distillation extractive est envoyé à une colonne destinée au stripping du solvant et disposée en aval de la dite colonne extractive, mais la colonne de stripping n'est pas forcément un appareil séparé de la colonne extractive; le dit procédé est **caractérisé en ce qu'**une solution aqueuse est dispersée dans une zone de mélange et introduite dans le produit de tête provenant à l'état de vapeur de la colonne de stripping du solvant, que la vapeur biphasée et saturée de gouttelettes de buée est directement circulée vers une zone de condensation, dans laquelle les deux phases de la vapeur passent à l'état liquide et l'émulsion condensée s'écoule dans une zone de séparation qui sert à la précipitation d'une partie de la phase liquide de l'émulsion, qui contient notamment les hydrocarbures purifiés, pour la séparer de l'autre partie de la phase liquide ayant une teneur importante en eau de solution avec les impuretés dissoutes dans l'eau.

2. Procédé selon la revendication No.1, **caractérisé en ce que** la séparation de l'une partie de la phase liquide de l'émulsion, qui contient notamment les hydrocarbures purifiés, de l'autre partie de la phase liquide, qui a une teneur importante en eau de solution avec des impuretés dissoutes dans l'eau, a lieu à l'aide d'un dispositif séparateur d'eau.

3. Procédé selon une des revendications 1 ou 2, **caractérisé en ce que**, pour le moins, une partie de l'eau de solvant séparée de l'émulsion est recyclée pour être introduite par dispersion, comme composant de la solution aqueuse, dans la dite zone de mélange et que la quantité résiduelle de l'eau de solvant séparée de l'émulsion est évacuée, en même temps que les dites impuretés, du cycle de l'eau de solvant pour être mise à la décharge.

4. Procédé selon une des revendications 1 à 3, **caractérisé en ce que**, pour le moins, une partie de la solution aqueuse introduite par dispersion dans la zone de mélange est de l'eau fraîche.

5. Procédé selon une des revendications 1 à 4, **caractérisé en ce que** la solution aqueuse introduite par dispersion dans la zone de mélange est mêlée avec un acide.

6. Procédé selon la revendication 5, **caractérisé en ce que** l'acide mêlé avec la solution aqueuse est de l'acide formique.

7. Procédé selon la revendication 5 ou 6, **caractérisé en ce que** les sels précipités dans la solution aqueuse au bout de l'apport de l'acide sont éliminés de la dite solution moyennant de précipitants.

8. Procédé selon les revendications 5 à 7, **caractérisé en ce que** l'apport de l'acide est réglé par la valeur pH.

9. Procédé selon une des revendications 1 à 8, **caractérisé en ce que** la solution aqueuse est refroidie avant d'être introduite par dispersion dans la zone de mélange.

10. Procédé selon une des revendications 1 à 9, **caractérisé en ce que** l'émulsion condensée dans la zone de condensation est surrefroidie avant d'être introduite dans la zone de séparation.

11. Dispositif pour la réalisation du procédé conformément, pour le moins, à une des revendications 1 à 10, **caractérisé en ce que** les zones de mélange et de condensation sont disposées dans une chambre commune enrobée et que la dite chambre est intégrée et enveloppée par la calandre d'un appareil individuel.
